Europäisches Patentamt

(19) European Patent Office

Office européen des brevets

(11) Veröffentlichungsnummer: **0 325 628 B1**

(12) **EUROPÄISCHE PATENTSCHRIFT**

(45) Veröffentlichungstag der Patentschrift:
21.11.91 Patentblatt 91/47

(51) Int. Cl.⁵: **A61K 31/60, A61K 9/08**

(21) Anmeldenummer: 88905833.5

(22) Anmeldetag: 19.07.88

(86) Internationale Anmeldenummer:
PCT/EP88/00649

(87) Internationale Veröffentlichungsnummer:
WO 89/00853 09.02.89 Gazette 89/04

(54) **SALICYLSÄUREHALTIGES MITTEL GEGEN SCHUPPENDE HAUTERKRANKUNGEN.**

(30) Priorität: 25.07.87 DE 3724691

(43) Veröffentlichungstag der Anmeldung:
02.08.89 Patentblatt 89/31

(45) Bekanntmachung des Hinweises auf die
Patenterteilung:
21.11.91 Patentblatt 91/47

(84) Benannte Vertragsstaaten:
AT BE CH DE FR GB IT LI LU NL SE

(56) Entgegenhaltungen:
EP-A- 0 063 870
DE-A- 762 726

(56) Entgegenhaltungen:
Pharm. Ind., Band 44, Nr. 6, 1982, U. Adams et
al.: "Vergleichende Untersuchung zur Wirk-
stoff-Freigabe von Salicylsäure aus Salben
mit mittelkettigen Triglyceriden sowie mit Paraffinkohlenwasserstoffen - in vitro und in vivo", Seiten 625-629, siehe Abschnitt 2:
"untersuchte Salben",; Tabelle 1

(73) Patentinhaber: Minninger, Konrad
Hattinger Strasse 45
W-5830 Schwelm (DE)

(72) Erfinder: TANG, David
Hauptstrasse 304
W-4690 Herne 2 (DE)
Erfinder: OBERHAGEMANN, Rainer
Hauptstrasse 304
W-4690 Herne 2 (DE)

(74) Vertreter: Weisse, Jürgen, Dipl.-Phys. et al
Bökenbusch 41 Postfach 11 03 86
W-5620 Velbert 11-Langenberg (DE)

## Beschreibung

Die Erfindung betrifft ein salicylsäurehaltiges Mittel zur lokalen Therapie von schuppenden Hauterkrankungen im Bereich der Human- und Veterinärmedizin. Zu diesen schuppenden Hauterkrankungen zählen beispielsweise Psoriasis, Tinea amiantacea und Seborrhoea sicca.

Es ist beispielsweise aus E. Mutschler, "Arzneimittelwirkungen", Wissenschaftliche Verlagsgesellschaft, Stuttgart 1975, S. 373, bekannt, daß Salicylsäure die Ablösung von Schuppen und die Erweichung von Hornmaterial bewirkt und in Form von Salicylvaseline, Salicylspiritus und Salicylkollodium zur Anwendung kommt. Als weitere Mittel zur Behandlung der vorerwähnten Hauterkrankungen sind unter anderem Salicylschweineschmalz, Salicyleucerin und Salicylgel in Gebrauch.

Darüberhinaus ist es auch bekannt, Lösungen von Salicylsäure in fetten Ölen wie Olivenöl, Rizinusöl und anderen zur Behandlung der vorgenannten Hauterkrankungen zu verwenden.

Weiterhin ist aus der EP-A-0063870 ein entzündungshemmendes Salbenpräparat für dermatologische Anwendungen bekannt, das allgemein ein entzündungshemmendes Mittel in einer Salbengrundlage enthält. Die Salbengrundlage besteht aus einem mittelkettigen Triglycerid, einem Carboxyvinylpolymer und Wasser im Gewichtsverhältnis (1-25) : (0,3-3) : (75-99).

Aus der DE-A-762726 sind Lösungsmittel bzw. Lösungsvermittler bekannt, mittels derer die Löslichkeit von Salicylsäure in Fetten und Ölen über 10% erhöht werden kann. Dazu zählen höhermolekulare aliphatische Alkohole und Alkoholgemische ($C_8$-$C_{14}$) wie Fettalkohole und Ether solcher Alkohole mit mehrwertigen Alkoholen, die wenigstens eine freie Hydroxylgruppe enthalten.

Nach der Veröffentlichung von U. Adams et al. (Pharm. Ind., Vol. 44, Nr. 6, 1982, Seiten 625 bis 629) wird Salicylsäure in Salben aufgenommen, die als Gele ausgebildet sind und als Salbengrundlage mittelkettige Triglyceride enthalten, die durch ein organisch modifiziertes Magnesiumschichtsilikat geliert werden. Solche Salbengele können Wollwachsalkohole als Emulgatoren enthalten und maximal 5,5 bzw. 5,7% Salicylsäure aufnehmen.

Im Gebrauch der bekannten salicylsäurehaltigen Mittel hat sich herausgestellt, daß diese bekannten Mittel bei ihrer Anwendung für die so behandelten Patienten aus einer Reihe von Gründen, zu denen beispielsweise eine erhebliche Geruchsbelästigung durch Ranzigwerden der fetten Öle während der Behandlung gehören, nicht oder nur wenig akzeptabel sind und in der Praxis den gewünschten Erfolg eigentlich nur bei stationärer Behandlung bringen. Das ist aber für die mit den Mitteln behandelten Patienten von besonderer Bedeutung, auch aus psychologischen Gründen.

Die Aufgabe der Erfindung besteht darin, ein salicylsäurehaltiges Mittel der eingangs genannten Art zu schaffen, das bei guter Verträglichkeit ohne nachteilhafte Wirkungen für den Patienten ist und eine nichtstationäre Behandlung ermöglicht.

Erfindungsgemäß wird diese Aufgabe dadurch gelöst, daß in dem Mittel 4 Gew.-% bis 10 Gew.-% Salicylsäure, 1 Gew.-% bis 7 Gew.-% eines aliphatischen 1,2-Diols und 83 Gew.-% bis 95 Gew.-% eines fetten Öls enthalten sind.

Die Sättigungskonzentrationen der Salicylsäure in fetten Ölen oder deren Mischungen liegt im Bereich von 3% bis maximal 5%, was für viele Anwendungsfälle zu niedrig ist. Überraschenderweise wurde nun gefunden, daß höhere Sättigungskonzentrationen erreicht werden können, wenn das erfindungsgemäße Mittel zusätzlich ein aliphatisches 1,2-Diol enthält. Dabei können verschiedene aliphatische 1,2-Diole zur Anwendung kommen, wobei sich 1,2-Propandiol und die verschiedenen Butylenglykole mit benachbarten Hydroxylgruppen als besonders günstig erwiesen haben.

Bei dem erfindungsgemäßen Mittel können gängige fette Öle, bevorzugt pflanzliche fette Öle, das sind Triglyceride eingesetzt werden. Besonders bewährt haben sich dabei die Triglyceride mittelkettiger Fettsäuren und deren Mischungen, wobei die Triglyceride der Caprylsäure, der Caprinsäure oder deren Mischungen, besonders bevorzugt werden. Die vorgenannten Verbindungen haben sich als besonders günstig erwiesen ; es lassen sich an Stelle der Triglyceride oder deren Mischungen aber auch andere Ester, zum Beispiel solche von ein- oder zweiwertigen Alkoholen und insbesondere mittelkettigen Fettsäuren, einsetzen.

Die erfindungsgemäßen Mittel sind auch bei Langzeitanwendung gut verträglich ; bisher wurden auch bei Langzeitanwendung keine allergischen Reaktionen beobachtet. Dabei wird eine hohe Wirksamkeit erreicht ; insbesondere wird schon nach relativ kurzer Anwendungszeit, je nach Schwere der Erkrankung, eine völlige Abschuppung, d.h. Erscheinungsfreiheit erreicht, die bei entsprechender Nachbehandlung erhalten bleibt.

Ein besonders wirkungsvolles, bevorzugtes Mittel nach der Erfindung enthält 4 bis 10 g Salicylsäure, 1 bis 7 g 1,2-Propandiol und 83 bis 95 g der Triglyceride von Caprylsäure, Caprinsäure oder deren Mischung.

Eine wichtige Ursache für die hohe Wirksamkeit der erfindungsgemäßen Mittel liegt möglicherweise darin, daß die gewählten Lösungsmittelgemische in therapeutisch besonders günstiger Weise auf die erkrankte Haut einwirken und dadurch einen sehr wirksamen Träger bilden, der die Salicylsäure an ihren Wirkungsort bringt.

2

Weiterhin ist die Behandlung mit den erfindungsgemäßen Mitteln nicht mit unerwünschten und inakzeptablen Belastungen für die Patienten verbunden, weil die erfindungsgemäßen Mittel rasch und vollständig in der Haut resorbiert werden. Es kommt daher nicht zu für den Patienten unerträglichen Geruchsbelästigungen durch Ranzigwerden der Fette oder fetten Öle und nicht zu den unerwünschten Verschmutzungen von Kleidung oder Bettwäsche. Ein weiterer ganz wesentlicher Vorteil der erfindungsgemäßen Mittel besteht darin, daß in den meisten Fällen ein Krankenhausaufenthalt nicht mehr erforderlich ist. Dadurch wurde insgesamt eine sehr hohe Akzeptanz durch die Patienten erreicht.

Bevorzugte Ausführungsbeispiele der Erfindung werden nachstehend im Zusammenhang mit ihrer Herstellungsvorschrift erläutert. Die dafür eingesetzten Stoffe genügten den Reinheitsanforderungen des D.A.B.

Salicylsäure wird in Mengen von 4, 5, 7.5 und 10 g abgewogen und jeweils mit einer Menge von 1 bzw. 2 bzw. 4.5 bzw. 7 g 1,2-Propandiol in üblicher Weise verrieben. Anschließend werden zu den 5, 7, 12 und 17 g der 1,2-Propandiol-Verreibung jeweils 95 bzw. 93 bzw. 88 bzw. 83 g eines pflanzlichen fetten Öls bzw. Triglycerids gegeben und die Mischung so lange gerührt, bis eine klare, ölige Lösung entsteht. Diese Mittel enthalten somit, jeweils in Gew.-% und bezogen auf das Gesamtgewicht = 100,

| Salicylsäure | 4 | 5 | 7.5 | 10 |
|---|---|---|---|---|
| 1,2-Propandiol | 1 | 2 | 4.5 | 7 |
| Triglycerid | 95 | 93 | 88 | 83 |

Bevorzugt wird dabei so verfahren, daß — ausgehend von der Sättigungskonzentration der Salicylsäure in dem Triglycerid — gerade so viel 1,2-Propandiol eingesetzt wird, daß eine gesättigte oder nahezu gesättigte Lösung der gewünschten Salicylsäuremenge in dem 1,2-Propandiol-Triglyceridgemisch erhalten wird. Jedenfalls hat es sich für die Behandlung als günstig erwiesen, wenn dafür gesättigte oder nahezu gesättigte Lösungen der Salicylsäure verwendet werden.

Diese Mittel wurden in der nachstehend beschriebenen Weise zur Behandlung von schuppenden Hauterkrankungen wie Psoriasis, Tinea Amiantacea, Seborrhoea sicca beim Menschen und von Sommer-Räude und sonstigen schuppenden Erkrankungen beispielsweise bei Ponys eingesetzt. Insgesamt hat sich bei diesen und anderen vergleichbaren Untersuchungen herausgestellt, daß unter dem Gesichtspunkt der Verträglichkeit, der Wirksamkeit und der Behandlungsdauer bis zur Erscheinungsfreiheit die Mittel, die 4 Gew.-% bis 7.5 Gew.-% Salicylsäure, 1 Gew.-% bis 4.5 Gew.-% des aliphatischen 1,2-Diols und 88 Gew.-% bis 93 Gew.-% des fetten Öls enthielten, die relativ beste Wirkung zeigten.

Bei weiteren Untersuchungen wurde gefunden, daß anstelle von 1,2-Propandiol auch die verschiedenen Butylenglykole mit benachbarten Hydroxylgruppen und gegebenenfalls auch andere aliphatische 1,2-Diole eingesetzt werden können. Ebenso können anstelle der pflanzlichen fetten Öle oder Triglyceride oder deren Mischungen Triglyceride von mittelkettigen Fettsäuren, das sind $C_8$- bis $C_{12}$-Säuren oder deren Mischungen, verwendet werden.

Die therapeutische Anwendung eines Ausführungsbeispiels, das 5 Gew.-% Salicylsäure, 2 Gew.-% 1,2-Propandiol und 93 Gew.-% mittelkettige Triglyceride (Gesamtgewicht des Mittels = 100) enthält, wird nachfolgend an Hand einer Patientengruppe von 150 Personen erläutert, die hauptsächlich an Psoriasis und zu einem kleineren Teil an anderen schuppenden Haut- und Kopfhauterkrankungen, z.B. seborrhoeischen Ekzemen litten. Die Behandlung bestand darin, daß

a)  bei Erkrankungen der Kopfhaut
das Mittel zwei- bis dreimal wöchentlich auf die befallenen Stellen der Kopfhaut aufgetragen wird ; die Behandlung kann unter Benutzung einer Dusch-Plastikhaube mit schließendem Gummirand und einer handelsüblichen Operationshaube zur Fixierung über Nacht erfolgen, und

b)  bei Erkrankungen am Körper
das Mittel ein- bis zweimal täglich auf die befallenen Stellen aufgetragen wird ; nach 10 bis 15 Minuten Einwirkungszeit ist das Mittel von der Haut aufgenommen.

Bei 8 Personen aus der Patientengruppe wurde die Behandlung vorzeitig abgebrochen aus Gründen, die nichts mit der eigentlichen Behandlung zu tun hatten. Bei 50 Personen aus der Patientengruppe wurde in Abhängigkeit von der Schwere der ursprünglichen Erkrankung totale Abschuppung und damit die erwünschte Erscheinungsfreiheit nach einer Behandlungszeit von

1    Monat bei leichter ursprünglicher Erkrankung,
bis zu 3  Monaten bei mittlerer ursprünglicher Erkrankung und
bis zu 4  Monaten bei schwerer ursprünglicher Erkrankung erzielt, wobei der Beginn der Abschuppung bereits in den ersten Wochen der Behandlung einsetzte.

Bei den meisten der restlichen 92 Patienten war die Abschuppung nach bis zu einjähriger Behandlungsdauer sehr weit fortgeschritten und läßt auch hier totale Abschuppung und damit die erwünschte Erscheinungsfreiheit erwarten. Allergische Reaktionen wurden nicht beobachtet, und die Behandlung wurde von den Patienten hervorragend akzeptiert. Dazu trägt sicher bei, daß das Mittel geruchsneutral ist und schnell in die Haut einzieht, wodurch keine Verschmutzung von Kleidung oder Bettwäsche erfolgt, sowie die Tatsache, daß nur bei besonderen Umständen und sehr schweren Erkrankungen ein Klinikaufenthalt erforderlich wird.

Als sehr vorteilhafte Nebenwirkung wurde deutlich erkennbarer vermehrter Haarwuchs, auch im veterinärmedizinischen Bereich festgestellt.

Die einmal erreichte Erscheinungsfreiheit kann auch in der Folgezeit aufrechterhalten werden. Dazu empfiehlt sich eine Folgetherapie unter Verwendung handelsüblicher, eine erneute Schuppenbildung reduzierender Mittel wie Dithranol im Wechsel mit den zuvor beschriebenen Mitteln.

**Patentansprüche**

1. Mittel enthaltend Salicylsäure und Ester von Fettsäuren, zur lokalen Therapie von schuppenden Hauterkrankungen im Bereich der Human- und Veterinärmedizin,
dadurch gekennzeichnet, daß das Mittel eine einphasige flüssige Lösung aus 4 Gew.-% bis 10 Gew.-% Salicylsäure, 1 Gew.-% bis 7 Gew.-% eines aliphatischen 1,2-Diols und 83 Gew.-% bis 95 Gew.-% eines fetten Öls bildet, jeweils bezogen auf das Gesamtgewicht des Mittels = 100.

2. Mittel nach Anspruch 1, dadurch gekennzeichnet, daß die aliphatischen 1,2-Diole aus der Gruppe 1,2-Propandiol und Butylenglykole mit benachbarten Hydroxylgruppen ausgewählt sind.

3. Mittel nach Anspruch 2, dadurch gekennzeichnet, daß das fette Öl aus der Gruppe pflanzlicher Triglyceride ausgewählt ist.

4. Mittel nach einem der Ansprüche 1 bis 3, dadurch gekennzeichnet, daß der Anteil an dem aliphatischen 1,2-Diol so gewählt ist, daß die Salicylsäure in wenigstens nahezu gesättigter Lösung vorliegt.

5. Mittel nach einem der vorstehenden Ansprüche, dadurch gekennzeichnet, daß das fette Öl wenigstens ein mittelkettiger Fettsäureester eines Alkohols ist, der aus der Gruppe einwertiger und zweiwertiger Alkohole und Glycerin ausgewählt ist.

6. Mittel nach Anspruch 5, dadurch gekennzeichnet, daß das fette Öl ein Triglycerid von mittelkettigen Fettsäuren ist.

7. Mittel nach Anspruch 6, dadurch gekennzeichnet, daß die mittelkettigen Fettsäuren aus Caprylsäure, Caprinsäure oder deren Mischung ausgewählt sind.

8. Mittel nach Anspruch 7, dadurch gekennzeichnet, daß das Mittel 4 bis 7.5 Gew.-% Salicylsäure, 1 bis 4.5 Gew.-% 1,2-Propandiol und 88 bis 95 Gew.-% des Triglycerids von Caprylsäure, Caprinsäure oder deren Mischung enthält, jeweils bezogen auf das Gesamtgewicht des Mittels = 100.

9. Mittel nach einem der vorstehenden Ansprüche, dadurch gekennzeichnet, daß die Konzentration des darin enthaltenen aliphatischen 1,2-Diols von 1 über 2 und 4,5 auf 7 Gew.-% und die Salicylsäurekonzentration von 4 über 5 und 7,5 auf 10 Gew.-% zunehmen, jeweils bezogen auf das Gesamtgewicht des Mittels = 100.

**Claims**

1. Agent containing salicylic acid and esters of fatty acids for local therapy of lepidosis in the field of human and veterinary medicine,
**characterized in that** the agent constitutes a one-phase liquid solution containing 4 to 10 percent of salicylic acid, 1 to 7 percent of an aliphatic 1,2-diol and 83 to 95 percent of a fatty oil, each in percent by weight of the agent.

2. Agent according to claim 1, **characterized in that** the aliphatic 1,2-diols are selected from the group consisting of 1,2-propane diol and butylene glycols having adjacent hydroxyl groups.

3. Agent according to claim 2, **characterized in that** the fatty oil is selected from the group of vegetable triglycerides.

4. Agent according to any one of claims 1 to 3, **characterized in that** the proportion of the alphatic 1,2-diol is selected such that the salicylic acid is present in at least almost saturated solution.

5. Agent according to any one of the preceding claims, **characterized in that** the fatty oil is at least one medium-chain fatty acid ester of an alcohol which is selected from the group consisting of monohydric and dihydric alcohols and glycerin.

6. Agent according to claim 5, **characterized in that** the fatty oil is a triglyceride of medium-chain fatty acids.

7. Agent according to claim 6, **characterized in that** the medium-chain fatty acids are selected from caprylic acid, capric acid or a mixture thereof.

8. Agent according to claim 7, **characterized in that** the agent contains 4 to 7.5 percent of salicylic acid, 1 to 4.5 percent of 1,2-propane diol and 88 to 95 percent of the triglyceride of caprylic acid, capric acid or a mixture thereof, each in percent by weight of the agent.

9. Agent according to any one of the preceding claims, **characterized in that** the concentration of the aliphatic 1,2-diol contained therein increases from 1 through 2 and 4.5 to 7 percent and the concentration of the salicylic acid increases from 4 through 5 and 7.5 to 10 percent, each in percent by weight of the agent.

## Revendications

1. Agent comprenant de l'acide salicylique et de l'ester d'acides gras, destiné à la thérapie locale d'affections de la peau écailleuses dans le domaine de la médecine humaine et vétérinaire, caractérisé par le fait que l'agent forme une solution liquide monophasée composée de 4% en poids à 10% en poids d'acide salicylique, 1% en poids à 7% en poids d'un 1,2-diol aliphatique et 83% en poids à 95% en poids d'une huile grasse, respectivement en référence au poids total de l'agent = 100.

2. Agent selon la revendication 1, caractérisé par le fait que les 1,2-diols aliphatiques sont choisis du groupe 1,2-diol de propane et glycols de butylène ayant des hydroxyles contigus.

3. Agent selon la revendication 2, caractérisé par le fait que l'huile grasse est choisie parmi le groupe des triglycérides végétaux.

4. Agent selon l'une des revendications 1 à 3, caractérisé par le fait que la part de 1,2-diol aliphatique est choisie de sorte que l'acide salicylique est présent en solution au moins approximativement saturée.

5. Agent selon l'une des revendications ci-dessus, caractérisé par le fait que l'huile grasse est au moins un ester d'acide gras à chaîne centrale d'un alcool choisi du groupe des alcools monovalents et bivalents et des glycérines.

6. Agent selon la revendication 5, caractérisé par le fait que l'huile grasse est un triglycéride d'acides gras à chaîne centrale.

7. Agent selon la revendication 6, caractérisé par le fait que les acides gras à chaîne centrale sont choisis parmi les acides capryliques, les acides capriques ou parmi un mélange de ceux-ci.

8. Agent selon la revendication 7, caractérisé par le fait que l'agent comprend 4 à 7,5% en poids d'acide salicylique, 1 à 4,5% en poids de 1,2-diol de propane et 88 à 95% en poids du triglycéride d'acide caprylique, d'acide caprique ou d'une mélange de ceux-ci, respectivement en référence au poids total de l'agent = 100.

9. Agent selon l'une des revendications ci-dessus, caractérisé par le fait que la concentration du 1,2-diol aliphatique contenu s'accroît de 1 via 2 et 4,5 à 7% en poids et la concentration de l'acide salicylique s'accroît de 4 via 5 et 7,5 à 10% en poids, respectivement en référence au poids total de l'agent = 100.